# EUROPEAN PATENT SPECIFICATION

(11) **EP 0 502 983 B1**
(45) Date of publication and mention of the grant of the patent: **28.08.1996**
(21) Application number: 91901116.3
(22) Date of filing: 03.12.1990
(51) Int. Cl.: G01N 33/48, G01N 33/86, G01N 21/78

(54) **METHOD OF MONITORING REAGENT DELIVERY IN A SCANNING SPECTROPHOTOMETER**
VERFAHREN ZUR ÜBERWACHUNG DER REAGENZFÖRDERUNG IN EINEM ABTASTSPEKTROPHOTOMETER
PROCEDE DE CONTROLE DE L'ALIMENTATION EN REACTIF DANS UN SPECTROPHOTOMETRE A BALAYAGE

(30) Priority: 01.12.1989 US 443953
(43) Date of publication of application: 16.09.1992
(73) Proprietor: Akzo Nobel N.V., NL-6824 BM Arnhem (NL)
(72) Inventor: DRISCOLL, Richard, Cornelius, Raleigh, NC 27614 (US); FISCHER, Timothy, J., Raleigh, NC 27604 (US)
(74) Representative: Hermans, Franciscus G.M.
(86) International application number: PCT/US90/07068
(87) International publication number: WO 91/08461

(56) References cited:
- GB-A- 2 039 035
- US-A- 3 059 524
- US-A- 4 204 839
- US-A- 4 329 149
- US-A- 4 409 334
- US-A- 4 506 626
- US-A- 4 543 335
- US-A- 4 849 340
- US-A- 4 911 549
- PATENT ABSTRACTS OF JAPAN vol. 7, no. 109 (P-196)(1254) 12 May 1983

## Description

### BACKGROUND OF THE INVENTION

The present invention relates to a method for monitoring reagent delivery in a scanning spectrophotometer and a reagent useful in such a method.

A problem in current reagent delivery systems is that once a reagent has been added to a reaction mixture, it is difficult to determine the actual amount of the reagent present in the reaction mixture. This is a particular problem in blood coagulation assays. In this type of assay, when a blood coagulating reagent such as thromboplastin or thrombin is aspirated into a pipette, air may be drawn into the pipette, so that an insufficient amount of the reagent may be dispensed.

Another problem with assays of the above type is that it may not be possible to easily determine whether there is a plasma sample to be tested present in the reaction cuvette.

### SUMMARY OF THE INVENTION

Therefore, it is an object of the present invention to provide a method for accurately measuring the amount of reagent dispensed into a reaction mixture.

It is another object of the invention to provide a method for determining whether the sample to be tested is present in the reaction mixture.

It is yet another object of the invention to provide a reagent useful in the above method.

The invention provides a method for measuring the concentration of a reagent in a reaction mixture including the steps of: adding dye to a reagent until the dye is at a known concentration in the reagent; mixing the reagent with a specimen to form a reaction mixture comprising a component which reacts with the reagent to form a reaction product; measuring the formation of a reaction product at a first spectral region; measuring the concentration of dye in the reaction mixture at a second spectral region in which the dye has an optical characteristic such as absorption or fluorescence, the second spectral region being different from the first spectral region, and determining the concentration of the reagent in the reaction mixture based on the concentration of dye measured. Variations caused by color or turbidity in the reagent or specimen are eliminated by normalizing using a measurement of optical characteristics of the reaction mixture at a third spectral region at which neither the reaction product nor the dye has an optical characteristic.

In another embodiment, the invention provides a blood coagulating reagent containing a dye which has an optical characteristic in a spectral region outside the reaction mixture's region of interest in order to monitor the concentration of the reagent.

### BRIEF DESCRIPTION OF THE DRAWINGS

Figure 1 illustrates the spectrum of Patent Blue VF, a dye useful in practicing the invention. Patent Blue VF (Sulphur Blue, CI42045) has an absorbance peak at 635nm and a secondary peak at 410nm, which are illustrated in the figure.

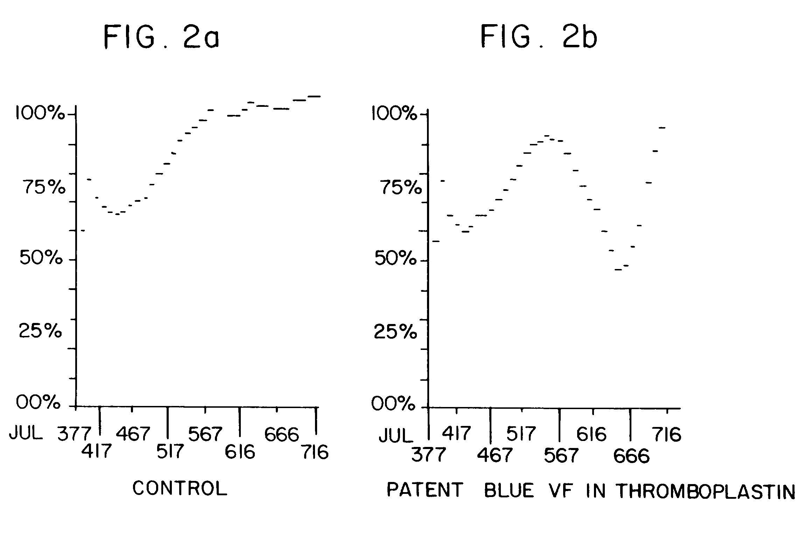

Figure 2 illustrates Patent Blue VF in a thromboplastin based PT clotting time reaction. Figure 2a shows the peak for the clot formed and Figure 2b shows the same reaction with dye in the reagent.

### DESCRIPTION OF THE PREFERRED EMBODIMENTS

The present method is preferably used in conjunction with an optical monitoring system such as that disclosed in concurrently filed WO-A-91 08455 , entitled "Multichannel Optical Monitoring System", assigned to the assignee of the present application. Various fluid reagents are mixed with the sample in a cuvette to form a reaction, the optical characteristics of which are monitored. The system is principally used to conduct tests on blood plasma, such as PT and APTT tests involving the addition of thromboplastin, thrombin, or CaCl₂ in order to convert fibrinogen to fibrin and form a clot.

In the present invention, a known amount of dye is added to a known amount of one of the reagents to be added to the reaction mixture. The concentration of dye in the reagent is preferably about 1 part per thousand. Preferably, the dye is added to blood coagulation reagent such as thromboplastin, thrombin or CaCl₂, but the dye may be added to other reagents in a clotting or other reaction.

The dye which is added to the blood coagulating reagent has the optical characteristic of either absorbing light at a given wavelength or fluorescing light of a given wavelength. Whichever optical characteristic the dye has, the dye should be compatible with the contents of the reaction mixture and have an optical characteristic in a spectral region that is outside the reaction mixture's region of interest, the region in which the measurement of the formation of reaction product is made.

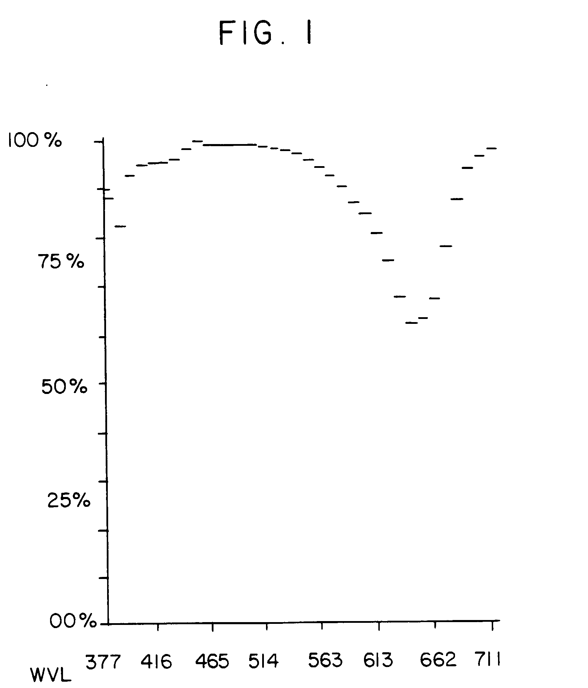

For example, when the reagent is a blood coagulating agent such a thrombin or thromboplastin and the specimen is blood plasma in a coagulation assay, an appropriate light absorbing dye, such as a blue dye, may be added to the thrombin before adding the thrombin to the plasma being tested. A preferred dye for this purpose is Patent Blue VF®, which has a spectrum as shown in Figure 1. As can be seen from this spectrum, Patent Blue VF has a large transmittance and therefore a low absorption in the range 400-550nm, the region in which clotting of blood in a coagulation assay is typically monitored by a spectrophotometer, but has a high absorption in the region of 635nm. While the exact range of absorption of the dye is not critical, preferably the dye does not absorb significantly in the region of 400-550nm and does absorb significantly in a range outside of this region.

By monitoring the concentration of dye in the reaction mixture at a wavelength at which it absorbs, the concentration of the thrombin in the reaction mixture can be determined. An indication of too much absorption at the dye's wavelength indicates that the thrombin has not been sufficiently diluted before adding the thrombin to the plasma or that there is no plasma present in the reaction mixture or present in the correct proportions. In contrast, too little absorption at the dye's wavelength indicates either that not enough thrombin has been added or that there is too much plasma present in the reaction mixture.

If either too much or too little absorption is measured by the spectrophotometer at the dye's wavelength, the spectrophotometer by means of a display or print-out warns the operator of the problem. By adding a suitable dye to any of the reagents and then monitoring the absorption at an appropriate wavelength for a short time after delivery of the reagent, the amount of reagent plus plasma can be estimated well enough to know whether the plasma and reagent are both present in the reaction mixture.

According to the present invention bichromatic techniques are used, wherein transmittance is measured in a third region, which is not affected by the dye or the reaction. By doing this all preanalytical variables can be eliminated to standardize the assay for variability among instruments, specimens and reagents, while still obtaining the advantage of measuring the concentration of the dye, and therefore the reagent, at the same time the reaction is taking place and the results simultaneously determined.

For assays using bichromatic techniques the preferred dye is Patent Blue VF (CI food blue 3, #42045), which has a primary peak at 635nm and a secondary peak at 410nm. Derivatives of Patent Blue VF or any other dyes may be used that meet the criteria of not interfering with the reaction (clot formation), having transmittance peaks at wavelengths that are not affected by the presence of plasma, not reacting with the therapeutic drugs (e.g. heparin) and being compatible with calcium chloride, thromboplastin, thrombin or any other reagent used in a particular assay.

Bichromatic techniques rely on the measurement of transmittance in three wavelength regions. The first measurement is taken at a wavelength affected by the particular assay. For example, clotting of blood is measured by absorption in the region of 400-550nm. The second measurement is taken in a region in which the dye absorbs light, but which is not affected by the reaction. For example, 635nm is the spectral peak for Patent Blue VF. The third measurement is taken in a spectral region that is not affected by the dye or the reaction. For blood coagulation assays we have taken the third measurement in the region of 705 to 715nm. Any other regions not affected by the reaction and the dye can be selected, however. In the above example, the third measurement could be taken below 400nm, such as at 250nm.

To standardize the determination of dye concentration transmittance characteristics of the specimen and reagent normalized using the formula: dye concentration = r(635nm)/r(705nm), where r = signal at reaction time zero/signal of water. By dividing the r value by the signal of water in the cuvette the determination will be corrected for instrument to instrument variation. By dividing r(635nm) by r(705nm) a correction is made in the calculation of dye concentration for interference and transmittance from turbidity or color density in the specimen or reagent. This is an adjustment for changes in available light, which in fact will adjust for any change in the light source or transmittance of any of the materials making up the reaction mixture.

In the preferred example for coagulation assays of blood plasma the absorbance is measured at a light wave length of 705nm to correct for such specimen variation. Similar results can be obtained within a range of wave lengths including 705nm, such as 700 to 715nm, as long as the wave length is one in which light is absorbed by the specimen. For example, the turbidity or light density of plasma, particularly from contaminants such as bilirubin, cause light in the approximate 705nm wave length range to be absorbed. Since the absorbance of the dye is in the 635nm range for a Patent Blue VF, for example, and the absorbance of the clot is in the 400 - 550nm range for these blood coagulation assays, adjusting for the absorbance at 705nm eliminates variability resulting from contaminants in the specimen.

While the above description has been of a specific reagent and reaction mixture, the invention may be practiced with other suitable reagents and reaction mixtures. Further, the addition of more than one reagent may be monitored by adding dyes having different principal absorption wavelengths to the reagents and monitoring the concentration of each of the dyes in the reaction mixture.

In a manner similar to that discussed above for a light absorbing dye, a dye which fluoresces light in a region outside the region of interest for the reaction mixture may be added to a blood coagulating agent. When a fluorescent dye is used instead of a light absorbing dye, the concentration of reagent is monitored by measuring the presence of light at a particular wavelength due to fluorescence. A suitable fluorescent dye for this purpose is rhodamine B which fluoresces in the red region of the spectrum. While the fluorescent dye preferably does not absorb light significantly in the reaction mixture's region of interest, it may absorb light at any wavelength of the spectrum.

### EXAMPLE I

### APTT Test:

Patent Blue VF, at a concentration of 8mg/L in CaCl₂, was compared to a control lot of CaCl₂ on the Coag-A-Mate X2 (Organon Teknika Corporation). A 1% increase in clotting time occurred when the dye was present, which was attributed to the fact that the X2 optics detect clot formation at approximately 620nm, which coincides with the dye transmittance. The CaCl₂ dye solution was also evaluated at 565nm. No significant difference was detected in the shape of the waveform at 565nm when the dye was present. The final concentration of dye in the sample was 3mg/L, equating to approximately 50% transmittance.

### EXAMPLE II

### PT Test:

Patent Blue VF was added to Thromboplastin at a concentration of 1.15mg/L resulting in a final sample concentration of 0.75mg/L. A 1% to 2% increase in the PT clotting time occurred when the dye was added to the thromboplastin. No significant difference was detected in the shape of the waveform at 565nm when the dye was present. Determination of clot formation was essentially unaffected by dye.

## Claims

1. A method for measuring the concentration of a reagent in a reaction mixture comprising the steps of:
adding dye to a reagent until the dye is at a given concentration in the reagent;
mixing the reagent with a sample to form a reaction mixture, wherein said sample comprises a component that reacts with the reagent to form a reaction product;
measuring the formation of reaction product at a first spectral region;
measuring the concentration of dye in the reaction mixture at a second spectral region in which the dye has an optical characteristic, the second spectral region being different from the first spectral region;
measuring the transmittance of the reaction mixture at a third spectral region in which neither the reaction product nor the dye have optical characteristics; and
determining the concentration of the reagent in the reaction mixture based on the concentration of the dye measured.

2. The method of claim 1, wherein the dye absorbs light in the second spectral region.

3. The method of claim 2, wherein the dye does not substantially absorb light in the first spectral region.

4. The method of claim 1, wherein neither the dye nor the reaction product substantially absorb light in the third spectral region.

5. The method of claim 1, wherein the reagent comprises thromboplastin and the sample comprises blood plasma.

6. The method of claim 1, wherein the reagent comprises thrombin and the sample comprises blood plasma.

7. The method of claim 1, wherein the reagent comprises CaCl₂ and the sample comprises blood plasma.

8. The method of claim 1, wherein the dye comprises a compound with the formula 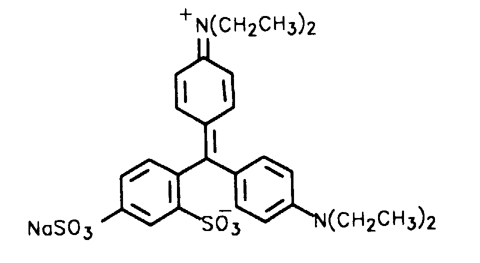 and the second spectral region is in the region of about 635 nm.

9. The method of claim 1, wherein the dye fluoresces light in the second spectral region.

10. The method of claim 9, wherein the dye comprises rhodamine B.

11. The method of claim 1, further comprising determining whether sample has been added to the added reaction mixture based on the concentration of dye in the reaction mixture.

12. The method of claim 1, wherein the added dye is at a concentration of about 1 part per thousand of the reagent.

13. A composition comprising a blood coagulation reagent containing a dye for monitoring of reagent or sample delivery, said dye having an optical charactenstic in a region outside of the range of about 400nm to 550nm.

14. The composition of claim 13, wherein said blood coagulation reagent comprises thrombin.

15. The composition of claim 13, wherein said blood coagulation reagent comprises thromboplastin.

16. The composition of claim 13, wherein said blood coagulation reagent comprises CaCl₂.

17. The composition of claim 13, wherein said dye absorbs light in a region outside of the range of about 400nm to 550nm.

18. The composition of claim 13, wherein said dye fluoresces in a region outside of the range of about 400nm to 550nm.

19. The composition of claim 13, wherein said dye comprises a compound with the formula

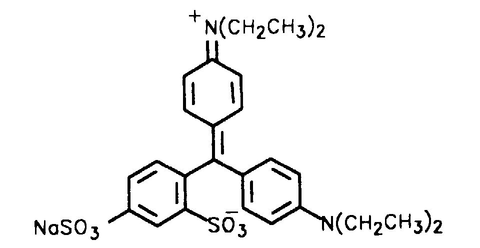

20. The composition of claim 18, wherein said dye comprises rhodamine B.

## Patentansprüche

1. Verfahren zum Messen der Konzentration eines Reagens in einem Reaktionsgemisch, welches folgende Schritte umfasst:
- Zugeben von Farbstoff zu einem Reagens, bis der Farbstoff im Reagens eine bestimmte Konzentration erreicht;
- Mischen des Reagens mit einer Probe, um ein Reaktionsgemisch zu bilden, worin diese Probe eine Komponente umfasst, die mit dem Reagens reagiert, um ein Reaktionsprodukt zu bilden;
- Messen der Reaktionsproduktbildung in einem ersten Spektralbereich;
- Messen der Farbstoffkonzentration im Reaktionsgemisch in einem zweiten Spektralbereich, in dem der Farbstoff eine optische Eigenschaft aufweist, wobei sich der zweite Spektralbereich vom ersten Spektralbereich unterscheidet;
- Messen der Durchlässigkeit des Reaktionsgemischs in einem dritten Spektralbereich, in dem weder das Reaktionsprodukt noch der Farbstoff optische Eigenschaften aufweisen; und
- Bestimmen der Konzentration des Reagens im Reaktionsgemisch basierend auf der gemessenen Farbstoffkonzentration.

2. Verfahren nach Anspruch 1, worin der Farbstoff im zweiten Spektralbereich Licht absorbiert.

3. Verfahren nach Anspruch 2, worin der Farbstoff im ersten Spektralbereich keine wesentlich Menge Licht absorbiert.

4. Verfahren nach Anspruch 1, worin weder der Farbstoff noch das Reaktionsprodukt im dritten Spektralbereich eine wesentliche Menge Licht absorbieren.

5. Verfahren nach Anspruch 1, worin das Reagens Thromboplastin und die Probe Blutplasma umfasst.

6. Verfahren nach Anspruch 1, worin das Reagens Thrombin und die Probe Blutplasma umfasst.

7. Verfahren nach Anspruch 1, worin das Reagens CaCl₂ und die Probe Blutplasma umfasst.

8. Verfahren nach Anspruch 1, worin der Farbstoff eine Verbindung der Formel 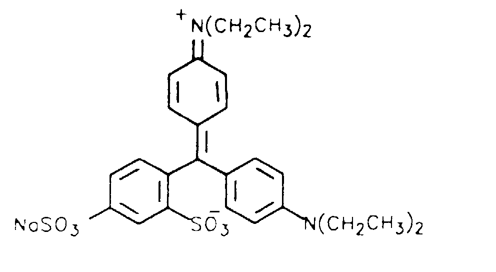 umfasst und der zweite Spektralbereich sich im Bereich von ungefähr 635nm befindet.

9. Verfahren nach Anspruch 1, worin der Farbstoff im zweiten Spektralbereich Licht fluoresziert.

10. Verfahren nach Anspruch 9, worin der Farbstoff Rhodamin B umfasst.

11. Verfahren nach Anspruch 1, welches weiter das Bestimmen, ob die Probe dem zugegebenen Reaktionsgemisch zugegeben wurde, umfasst, und zwar auf der Basis der Farbstoffkonzentration im Reaktionsgemisch.

12. Verfahren nach Anspruch 1, worin der zugegebene Farbstoff eine Konzentration von ungefähr 1 Tausendstel des Reagens aufweist.

13. Zusammensetzung, welche ein Blutkoagulierungsreagens, das einen Farbstoff enthält, um die Reagens- oder Probenabgabe zu überwachen, umfasst, wobei dieser Farbstoff eine optische Eigenschaft in einem Bereich ausserhalb des Bereichs von ungefähr 400nm bis 550nm aufweist.

14. Zusammensetzung nach Anspruch 13, worin dieses Blutkoagulierungsreagens Thrombin umfasst.

15. Zusammensetzung nach Anspruch 13, worin dieses Blutkoagulierungsreagens Thromboplastin umfasst.

16. Zusammensetzung nach Anspruch 13, worin dieses Blutkoagulierungsreagens CaCl₂ umfasst.

17. Zusammensetzung nach Anspruch 13, worin dieser Farbstoff in einem Bereich ausserhalb des Bereichs von ungefähr 400nm bis 550nm Licht absorbiert.

18. Zusammensetzung nach Anspruch 13, worin dieser Farbstoff in einem Bereich ausserhalb des Bereichs von ungefähr 400nm bis 550nm fluoresziert.

19. Zusammensetzung nach Anspruch 13, worin dieser Farbstoff eine Verbindung der Formel 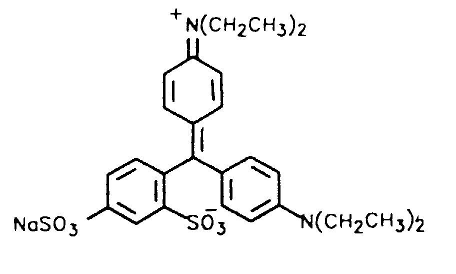 umfasst.

20. Zusammensetzung nach Anspruch 18, worin dieser Farbstoff Rhodamin B umfasst.

## Revendications

1. Un procédé de mesure de la concentration en réactif dans un mélange réactionnel comprenant les étapes consistant:
- à ajouter un colorant à un réactif jusqu'à ce que le colorant atteigne une concentration prédéterminée dans le réactif;
- à mélanger le réactif avec un échantillon pour former un mélange réactionnel, ledit échantillon comprenant un constituant qui réagit avec le réactif pour former un produit réactionnel;
- à mesurer la formation du produit réactionnel dans un premier domaine spectral;
- à mesurer la concentration du colorant dans le mélange réactionnel dans un deuxième domaine spectral dans lequel le colorant présente des caractéristiques optiques, le deuxième domaine spectral étant différent du premier domaine spectral;
- à mesurer le facteur de transmission du mélange réactionnel dans un troisième domaine spectral dans lequel ni le produit réactionnel, ni le colorant, ne possèdent des caractéristiques optiques, et
- à déterminer la concentration du réactif dans le mélange réactionnel à partir de la mesure de la concentration du colorant mesurée.

2. Le procédé de la revendication 1, dans lequel le colorant absorbe de la lumière dans le deuxième domaine spectral.

3. Le procédé de la revendication 2, dans lequel le colorant n'absorbe pas sensiblement la lumière dans le premier domaine spectral.

4. Le procédé de la revendication 1, dans lequel ni le colorant, ni le produit réactionnel n'absorbent sensiblement la lumière dans le troisième domaine spectral.

5. Le procédé de la revendication 1, dans lequel le réactif comprend la thromboplastine et l'échantillon comprend du plasma sanguin.

6. Le procédé de la revendication 1, dans lequel le réactif comprend la thrombine et l'échantillon comprend du plasma sanguin.

7. Le procédé de la revendication 1, dans lequel le réactif comprend du CaCl₂ et l'échantillon comprend du plasma sanguin.

8. Le procédé de la revendication 1, dans lequel le colorant comprend un composé de formule: 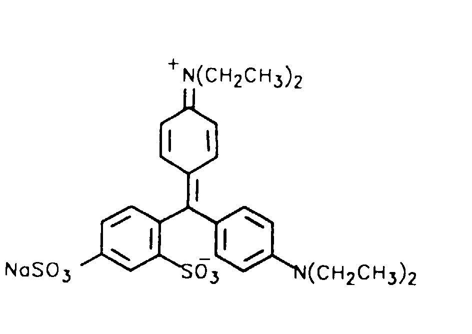 et le deuxième domaine spectral est dans la région au voisinage de 635 nm.

9. Le procédé de la revendication 1, dans lequel le colorant émet de la lumière par fluorescence dans le deuxième domaine spectral.

10. Le procédé de la revendication 9, dans lequel le colorant comprend la rhodamine B.

11. Le procédé de la revendication 1, comprenant en outre une étape permettant de déterminer l'ajout éventuel de l'échantillon au mélange réactionnel additionné, d'après la concentration de colorant dans le mélange réactionnel.

12. Le procédé de la revendication 1, dans lequel le colorant ajouté présente une concentration d'environ 1 partie pour mille du réactif.

13. Une composition comprenant un réactif de coagulation du sang renfermant un colorant pour le contrôle de l'alimentation en réactif ou en échantillon, ledit colorant présentant des caractéristiques optiques dans une région située hors de la région d'environ 400 nm à 550 nm.

14. La composition de la revendication 13, dans laquelle ledit réactif de coagulation sanguine comprend de la thrombine.

15. La composition de la revendication 13, dans laquelle ledit réactif de coagulation sanguine comprend de la thromboplastine.

16. La composition de la revendication 13, dans laquelle ledit réactif de coagulation sanguine comprend du CaCl₂.

17. La composition de la revendication 13, dans laquelle ledit colorant absorbe de la lumière dans une région située hors de la région d'environ 400 nm à 550 nm.

18. La composition de la revendication 13. dans laquelle ledit colorant présente une fluorescence dans une région située hors de la région d'environ 400 nm à 550 nm.

19. La composition de la revendication 13, dans laquelle ledit colorant comprend un composé de formule:

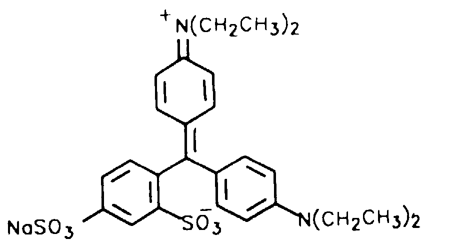

20. La composition de la revendication 18, dans laquelle ledit colorant comprend de la rhodamine B.
